Europäisches Patentamt

European Patent Office

Office européen des brevets

(11) Publication number: 0 216 564
A2

# EUROPEAN PATENT APPLICATION

(21) Application number: 86306950.6

(22) Date of filing: 09.09.86

(51) Int. Cl.⁴: **C 12 N 15/00**
C 12 N 7/00, A 61 K 39/21,
C 12 N 1/20
//(C12N1/20,C12R1:19)

(30) Priority: 09.09.85 US 774040  18.07.86 US 887614

(43) Date of publication of application:
01.04.87 Bulletin 87/14

(84) Designated Contracting States:
AT BE CH DE FR GB IT LI LU NL SE

(71) Applicant: CETUS CORPORATION
1400 Fifty-Third Street
Emeryville California 94608 (US)

(72) Inventor: Nunberg, Jack Henry
5933 Chabot Road
Oakland California 94618 (US)

Gilbert, James Harris
3296 Jordan Road
Oakland California 94602 (US)

(74) Representative: Bizley, Richard Edward et al
BOULT, WADE & TENNANT 27 Furnival Street
London EC4A 1PQ (GB)

The applicant has filed a statement in accordance with Rule 28 (4) EPC (issue of a sample only to an expert)

(54) **Infectious recombinant virus, vaccines containing said virus for feline leukemia, and methods for the production thereof.**

(57) Infectious recombinant viruses that contain a chimeric FeLV envelope gene and are useful as feline leukemia vaccines are described. The viruses are made by transfecting susceptible mammalian cells with DNA of the virus and an insertion vector that includes a chimeric gene comprising a promoter that is active in the cells. a translation start codon, and a DNA sequence that encodes an FeLV envelope protein and is under the control of the promoter. Vaccines composed of such infectious recombinant viruses and a parenteral vehicle or of inactivated preparation of cells or cell fractions which had been infected with said recombinant viruses are also described.

## Description

## INFECTIOUS RECOMBINANT VIRUS, VACCINES CONTAINING SAID VIRUS FOR FELINE LEUKEMIA, AND METHODS FOR THE PRODUCTION THEREOF

This invention is in the fields of recombinant DNA technology and immunoprevention of feline leukemia. More specifically, it relates to infectious recombinant virus vaccines for feline leukemia that raise an immune response to the viral envelope protein of feline leukemia virus (FeLV).

UND> >FeLV

FeLVs are a group of contagious oncogenic RNA viruses that cause both neoplastic and non-neoplastic diseases in cats. Infected cats are often immunosuppressed and many succumb to secondary infections. [Hardy et al., Feline Leukemia Virus (1980).] FeLV infections are the main cause of disease-related deaths in cats. During FeLV replication, a DNA copy of the viral RNA genome is made and inserted into the DNA of infected cells. The integrated FeLV DNA codes for the replication of more virus which is shed from infected cells. By recombining with the host's genes to generate feline sarcoma virus (FeSV), the virus can cause cell transformation.

The FeLV genome is a 60-70S single-stranded RNA dimer consisting of a gag gene that encodes the internal viral proteins, a pol gene that codes for the viral RNA dependent DNA polymerase (reverse transcriptase), and an env gene that encodes the viral envelope proteins gp70 and p15E. The FeLV env gene encodes a precursor glycoprotein, gp85, which is proteolytically processed to yield the mature gp70 and p15E proteins. These proteins are complexed via disulfide linkages and are found on the membranes of infected cells and virions. The major envelope glycoprotein gp70 is involved in host-receptor recognition and binding.

Most membrane proteins contain a hydrophobic region which is believed to insert within the lipid membrane and thereby anchor the protein to the membrane. The hydrophobic p15E protein is thought to be such a membrane-anchoring protein. Deletion of such regions from the vesicular stomatitis virus (VSV) G protein and herpes simplex gD protein results in molecules which are no longer bound to the membrane and are secreted into the cell culture supernatant [Rose et al., Cell, 30:753-762 (1982); Laskey et al., Bio/Technology, 2 (6):527-532 (1984), respectively]. In the development of immunoglobin-expressing cells, IgM expression progresses from a membrane-bound to secreted form of protein. Such change in form occurs via an RNA splicing event which deletes a hydrophobic transmembrane segment from the IgM protein [Alt et al., Cell, 20:293-301 (1980); Early et al., Cell, 20:313-319 (1980)].

The p15E protein of the FeLV env gene has been implicated in the immunosuppression associated with FeLV disease (Mathes et al., Nature, 274:687-689 (1978)]. Inactivated FeLV has been reported to inhibit lymphocyte proliferation in vitro [Hebebrand et al., Cancer Research, 37:4532-4533 (1977)], and this effect has been attributed to the FeLV p15E protein (Mathes et al., id.].

The DNA sequence of the env gene is described in J Virology (1983) 46:871-880. PCT/US/84/01963 (published 18 December 1985) illustrates the nucleotide sequence of the coding strand of the env gene of the Gardner-Arnstein (GA) strain of FeLV, and the amino acid sequence deduced therefrom. Viral interference and neutralization tests have shown that there are three subgroups of envelope antigens (designated A, B and C) that are similar but distinct from each other and give rise to the three recognized FeLV subgroups (also designated A, B and C).

Subgroup A virsues represent the ecotropic members of this virus group. Viruses of subgroup B are believed to derive from in vivo recombination events between the ecotropic subgroup A virus and endogenous FeLV-like sequences within the cat genome. [Stewart et al., J. Virol., 58(3):825-834 (June, 1986).] The subgroups differ in their natural distribution in that subgroup B and C viruses are found only in association with the subgroup A virus. [Jarrett et al., int. J. Cancer, 21:334-337 (1978).] Although common antigenic determinants exist between viruses of different subgroups, specific differences with regard to infectivity and disease progression have also been noted. [Jarrett et al., Int. J. Cancer, 21:466-472 (1978).] That the subgroup A virus is critical in the transmission of infection has been demonstrated by the observation that this virus is found in all FeLV-viremic animals; whereas subgroup B and/or C virus are found in approximately 50% of animals, but only in association with subgroup A virus [Jarrett et al., Int. J. Cancer, 21:334-337 (1978).]

The fate of a cat that is exposed to FeLV will depend upon its immune response to the FeLV -- particularly to the FeLV envelope antigens. Studies have shown that about 40% of the exposed population produce high titers of anti- FeLV envelope antibodies and become immune, about 30% do not respond adequately and become persistently infected, and about 30% are neither infected nor immunized but remain susceptible. The fact that a significant portion of the exposed population acquires natural immunity has led investigators to try a variety of materials as vaccines against FeLV. Inactivated virus was found to be ineffective except at high doses. Purified gp70 was also reported to be generally ineffective as a vaccine. Killed tumor cells have been reported to be successful in preventing leukemia but not viremia. A soluble tumor cell antigen obtained from the tissue culture medium of an FeLV transformed cell line (FL-74) was also tried and found effective in preventing the induction of FeLV virus infection. PCT/US 84/01963, published 18 December 1985 describes FeLV vaccines based on recombinant, microbially produced FeLV proteins.

Live, Recombinant Virus Vaccines

Moss, B., et al [Methods in Gene Amplification, (Elsevier-North Holland) Volume 3, pages 202-213 (1983); PNAS (USA) (1982) 79:7415-7419; PNAS (USA) (1983) 80:7155-7159; J Virology (1984) 49:857-864; and PCT/US85/01863, published June 7, 1984, International Pub. No. WO 84/02077] describe the insertion of heterologous genes into the genome of vaccinia virus. These genes are then expressed during viral replication within the host resulting in an immune response to these gene products, as well as to vaccinia. Using this strategy, significant immunological response to and/or protection against challenge from a variety of pathogens, including influenza [PNAS (1983) 80:7155; Nature (1984)311:578], herpes simplex [Science (1985) 228:737], hepatitis B [Moss et al., Nature (1984) 311:67], and Plasmodium knowlesi [Science (1984) 224:397] has been demonstrated.

The technique involves construction of a plasmid insertion vector containing the heterologous gene downstream from a vaccinia viral promoter all of which is inserted into the vaccinia thymidine kinase (TK) gene contained in the insertion vector. Co-transfection of vaccinia DNA and the insertion vector into vaccinia virus-infected cells allows for homologous recombination between the TK sequences in the viral DNA and the plasmid, resulting in the insertion of the heterologous gene into the vaccinia genome, and interruption of the viral TK gene. Recombinant viruses can be selected for by virtue of their TK-minus phenotype.

Live, recombinant Herpes Simplex I (HSV I) virus has been constructed for a variety of purposes by Roizman, et al. [Cell (1981) 25:227-232; Cell (1981) 24:555-565; Cell (1980) 22:243-255, Dev. Biol. Standardization (1982) 52:287-304, and European Patent 074 808 A2 (1982)] using methods first developed for use in the herpesvirus genetic research and similar to those later employed in the vaccinia virus work described above. Specific alterations in HSV I genes can be engineered on bacterial plasmids in vitro, and these sequences can then be inserted into the HSV I genome via homologous recombination in cells transfected to contain viral genomic and plasmid sequences. Using this method, and a strategy analogous to that used in the construction of live, recombinant vaccinia viruses, Shih et al. [PNAS (1984) 81:5867-5870] have reported the construction of a live, recombinant HSV I virus expressing an inserted engineered hepatitis B surface antigen (HBSAg) gene; the HBSAg gene was engineered for expression using an HSV promoter and was inserted into the TK gene of HSV I in a bacterial plasmid and used to construct a live, recombinant HSV I virus expressing the HBSAg gene.

Infectious recombinant viruses created by the methods of the instant invention are useful as feline leukemia virus vaccines. Such vaccines can be administered by a variety of routes, for example, parenterally (subcutaneously, intramuscularly, intraorbitally, intracapsularly, intraspirally, intravenously, intrasternally, among others), by intranasal aerosol, by scarification, or orally. The preferred route of aministration when a recombinant feline herpesvirus (FHV) is employed is by subcutaneous or intramuscular injection or by intranasal aerosol. When a recombinant vaccinia virus is used, the preferred administration route is by scarification or by intramuscular or subcutaneous injection.

Inactivated Subunit Vaccines

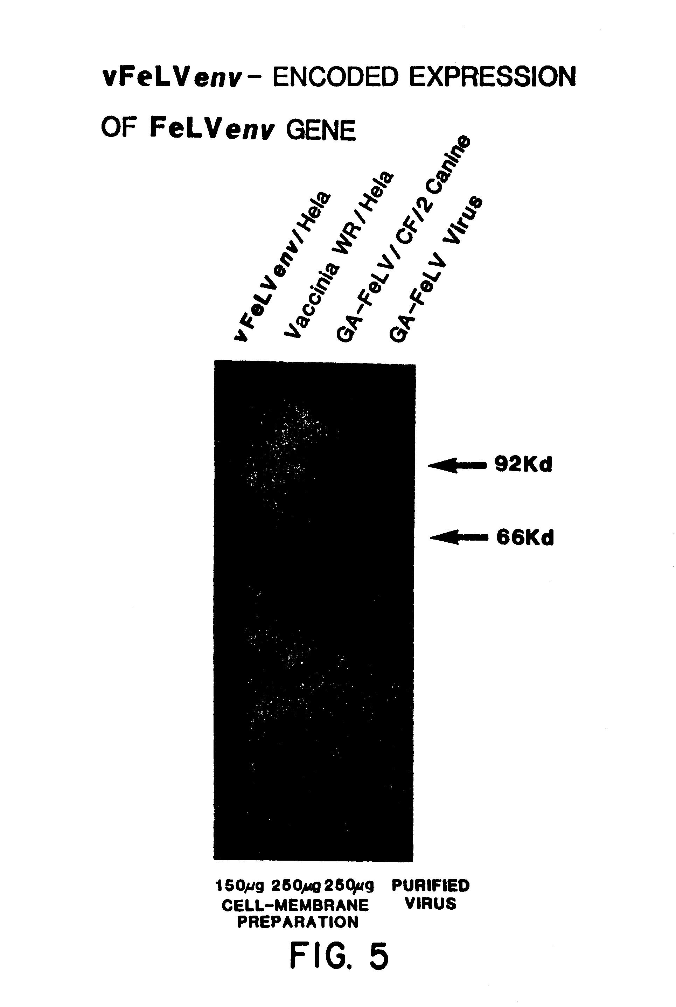

The vaccine can be composed essentially of the recombinant viruses themselves with a parenteral vehicle or can be derived from preparations of cells infected with the recombinant virus, which are inactivated by, for example, treatment with formalin or b-propiolactone (BPL). Human Hela cells infected with recombinant vaccinia virus vFeLVenv have been shown to accumulate on their surfaces substantially more FeLV gp70 than do cells infected with authentic FeLV virus. [See Figure 5 and discussion below in Example 3(B) (2).] The entire culture of infected cells, or semi-purified fractions thereof, for example, a membrane fraction, can be inactivated. for example, with formalin, BPL among other methods known in the art, and can be used as a subunit vaccine to raise antibodies against FeLV gp70 and thereby protect cats against FeLV infection.

Crude preparations of membrane proteins can be isolated from virus-infected cells. An example of such a crude membrane preparation is described under the Materials and Methods section below in the first paragraph of the subsection thereof entitled Immunological Analysis of FeLV env Protein Expression.

The present invention provides live, recombinant viruses that encode the expression of an FeLV envelope protein, means for making such viruses, and FeLV vaccines based on said viruses or in inactivated cell preparations infected with said virsuses.

Accordingly, one aspect of the invention is an insertion vector for use in transfecting mammalian cells to produce a recombinant form of a virus that encodes the expression of an immunogenic feline leukemia virus envelope protein, said vector comprising:

(a) a replicator;

(b) at least a portion of a nonessential region of the genome of said virus; and

(c) a chimeric gene inserted within said nonessential region comprising a promoter that is active in cells infected with the virus, a translation start codon, and a DNA sequence that codes for said protein and is under the control of said promoter.

Another aspect of the invention is an infectious recombinant virus useful in or for preparing a feline leukemia virus vaccine, said virus having a chimeric gene comprising a promoter that is active in feline cells infected with the virus, a translation start codon, and a DNA sequence that codes for an immunogenic feline leukemia virus envelope protein and is under the control of said promoter, inserted into a nonessential region of the genome of said virus.

Still another aspect of the invention is a host mammalian cell infected with the above-described recombinant virus.

Another aspect of the invention is FeLV vaccine comprising inactivated preparations of cells or fractions thereof which had been infected with the above-described recombinant virus, wherein said fractions contain a sufficient amount of an immunogenic feline leukemia virus envelope protein to generate an immune response against said protein. Said inactivated cell preparations can further comprise a pharmaceutically acceptable parenteral carrier.

Another aspect of the invention is a FeLV vaccine comprising a sufficient amount of the above-described recombinant virus to generate upon vaccinal infection and replication in the host an immune response against said protein and a pharmaceutically acceptable parenteral vehicle.

Yet another aspect of the invention is use of the vectors of the invention in producing a vaccine against feline leukemia virus.

Method aspects of the invention include:-

(A) A method of producing an insertion vector for use in transfecting mammalian cells to produce a recombinant form of a virus that encodes the expression of an immunogenic feline leukemia virus envelope protein, which comprises providing, linked together,

(a) a replicator;

(b) at least a portion of a nonessential region of the genome of said virus; and

(c) a chimeric gene inserted within said nonessential region comprising a promoter that is active in cells infected with the virus, a translation start codon, and a DNA sequence that codes for said protein and is under the control of said promoter.

(B) A method of producing an infectious recombinant virus for use in a feline leukemia virus vaccine including the step of transfecting a cell with virus DNA and an insertion vector comprising

(a) a replicator;

(b) at least a portion of a nonessential region of the genome of said virus; and

(c) a chimeric gene inserted within said nonessential region comprising a promoter that is active in cells infected with the virus, a translation start codon, and a DNA sequence that codes for said protein and is under the control of said promoter.

(C) A method for making a feline leukemia virus vaccine which comprises formulating a virus having a chimeric gene comprising a promoter that is active in cells to be infected with the virus, a translation start codon, and a DNA sequence that codes for an immunogenic feline leukemia virus envelope protein and is under the control of said promoter, said chimeric gene being inserted into a nonessential region of the genome of said virus, and/or a cell carrying such virus with a suitable carrier, adjuvant or vehicle.

In the drawings:

Figure 1 is a flow diagram of the derivation of the insertion vector, pVGA, containing the FeLV envelope gene and vaccinia promoter flanked by the vaccinia virus TK gene.

Figure 2 is a diagram of the GA- FeLV B proviral genome, and an exploded view of the envelope gene fragment used in Example 1. In the exploded view are restrictions sites which can be used to make expression plasmids of this invention as prepared according to Example 4 below.

Figure 3 is a flow diagram of the derivation of the plasmid pGApv containing the FeLV envelope gene.

Figure 4 is a flow diagram of the generation of the recombinant vaccinia virus, vFeLVenv.

Figure 5 shows the results of an immunologic analysis (Western blot) of the FeLV envelope gene product encoded by the recombinant vaccinia virus vFeLVenv and that encoded by authentic GA-FeLV B. The lanes represent: (1) cell membrane protein (150 µg) from Hela cells infected with vFeLVenv; (2) cell membrane protein (250 µg) from Hela cells infected with WR vaccinia virus; (3) cell membrane protein (250 µg) from canine CF/2 cells expressing authentic molecularly cloned GA-FeLV-$\beta$ virus; and (4) partially purified GA-FeLV virus from culture supernatants of CF/2 cells.

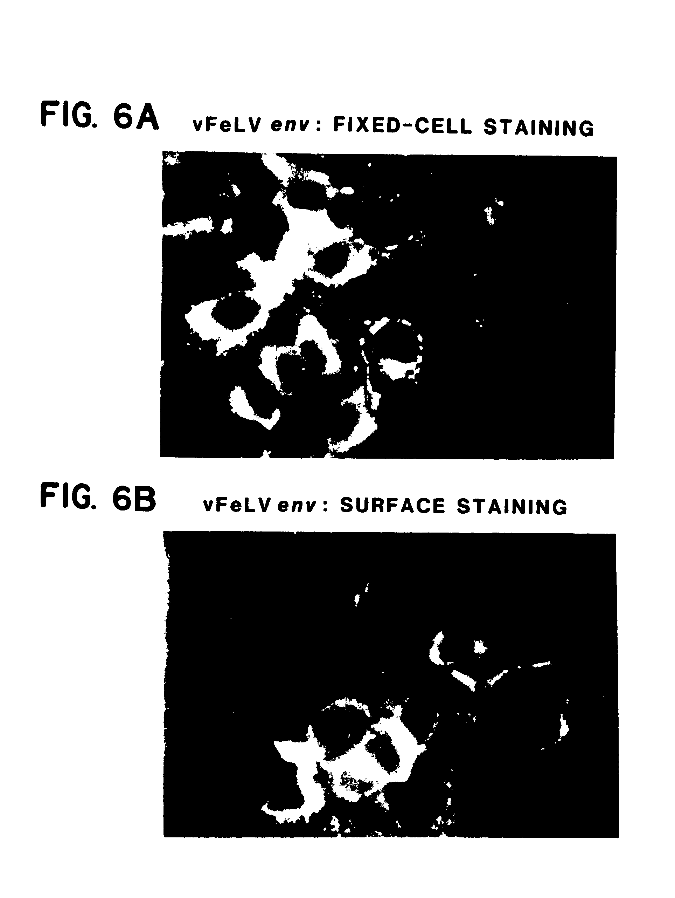

Figure 6 shows the results of an immunofluorescent analysis of cells infected with vFeLVenv (panels A and B). Hela cell monolayers were infected with the recombinant vaccinia virus vFeLV env at a low multiplicity of infection and examined by indirect immunofluorescence 24 hours after infection. FeLV env protein was detected using a FeLV gp70-specific monoclonal antibody. Panel A shows whole cell immunofluorescence in cells fixed and permeabilized in cold acetone. Panel B shows live cell immunofluorescence in which only FeLV env protein expressed on the surface of infected cells is detected. Exposure times for the photomicrographs were 0.5 min and 2 min, respectively.

Figure 7 compares the results of immunoprecipitation experiments with cells infected with recombinant vaccinia virus vFeLVenv and with those obtained from cells persistently infected with authentic GA-FeLV. (Surface Immunoprecipitation was from $1 \times 10^7$ cells whereas Total Cell Lysate Immunoprecipitation was from $2.5 \times 10^6$ cells.) Lanes are as follows: (1) uninfected Hela cells; (2) Hela cells infected with WR virus; (3) Hela cells infected with vFeLVenv virus; and (4) human RD cells infected with GA-FeLV.

Figure 8 illustrates an immunoblot analysis showing vFeLVenv-encoded env protein expression in human, murine and feline cells. Comparable cell monolayers of human Hela cells, murine L929 cells, and feline CRFK cells were infected with recombinant vFeLV env virus, or with WR vaccinia virus. Membrane preparations (100 ug protein) were analyzed to determine FeLV env protein.

Figure 9 is a flow chart of the procedure for making the plasmid ptGAΔHgiAI.

Figure 10 shows a preferred insertion vector, pVGA-VSVG, utilizing the vesicular stomatitis virus (VSV) protein G transmembrane region, as an alternative to the p15E anchor region of the FeLV envelope gene.

## Modes for Carrying Out the Invention

As used herein, the term "FeLV envelope protein" is intended to include the entire envelope protein (both the gp70 and the p15E), the gp70 protein, the gp70 envelope protein of FeLV and the transmembrane region from an alternative source, or immunogenic fragments thereof. The term "FeLV envelope sequence" is defined as the nucleic acid sequence encoding for the FeLV envelope protein, as just defined. The terms are not limited to any subgroup or strain.

As used herein, the phrase "nonessential region of the genome" is intended to denote a DNA sequence of the viral genome that is not essential for viral infectivity or replication.

Various plasmids, cosmids, or phages may be used to make the insertion vector. Once constructed, the vector contains a DNA sequence that encodes a FeLV envelope protein and a translation start codon downstream from a promoter that is active in host mammalian cells infected with the virus. Viral promoters are preferred, particularly those that are present in the native genome of the virus to be engineered. The FeLV envelope sequence/start codon/viral promoter construct is sometimes referred to herein as "an FeLV envelope gene expression cassette". The vector also includes a nonessential region of the viral genome of the virus to be engineered. The cassette is inserted into the nonessential region. Preferably the nonessential region includes a marker function that permits selection of recombinants. That is, interruption of the region by insertion of the cassette causes a detectible phenotypic change. A preferred nonessential region is the thymidine kinase (tk) gene. This region is excised from the viral genome, purified, and inserted into the vector that is to receive the cassette. Convenient restriction sites in the region are used to insert the cassette.

Example 1 below details the construction of a recombinant vaccinia virus encoding the expression of the feline leukemia virus (FeLV) envelope gene of the Gardner-Arnstein (GA) strain of FeLV subgroup B. Example 2 describes the analogous construction wherein the FeLV envelope gene of a FeLV subgroup A virus is encoded. The latter construction expressing the subgroup A FeLV envelope gene is preferred. Example 3 characterizes by a number of different types of experiments the FeLV envelope gene expression by the recombinant vaccinia virus vFeLVenv of subgroup B constructed according to Example 1.

Example 4 illustrates a further alternative construction for an infectious recombinant virus vaccine for feline leukemia wherein the p15E transmembrane region of FeLV of Examples 1 and 2 is replaced with the hydrophobic transmembrane region of the vesicular stomatitis virus (VSV) G protein. The Example 4 construction represents a preferred construction.

As indicated in the Background, supra, the p15E membrane anchoring protein of FeLV has been implicated in the immunosuppression associated with FeLV disease. Riedel [J. Virol., 54:224-228 (1985)] has shown that the hydrophobic transmembrane region of the vesicular stomatitis virus (VSV) G protein or of the fowl plague virus hemagglutinin are able to direct another virus protein, the Semliki Forest virus E2 protein, to the cell surface. Therefore, to enhance the immunogenicity of FeLV vaccines, it is preferred that the infectious recombinant viruses prepared according to this invention contain a gene encoding for a transmembrane protein other than the p15E protein of FeLV. Hydrophobic transmembrane regions from many different sources can be used to replace p15E functionally in the construction of Examples 1 and 2 below. For example, other transmembrane proteins include the red blood cell protein glycophorin and the coat proteins of filamentous bacteriophage. It is preferred that such hydrophic transmembrane regions be from the VSV G protein, herpes simplex gD protein, immunoglobin M or G proteins, or from the fowl plague virus.

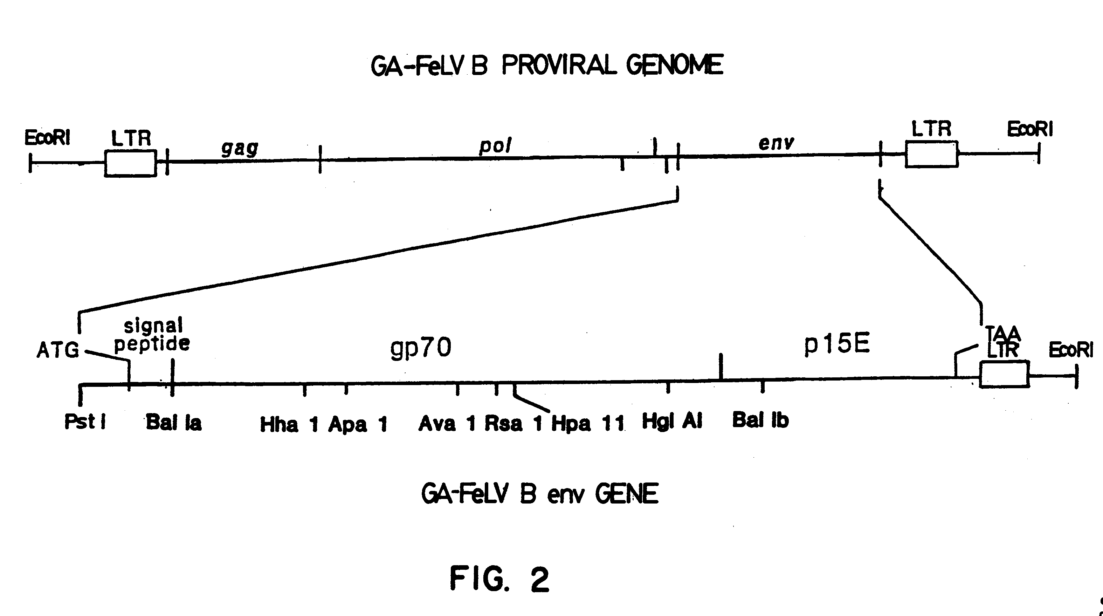

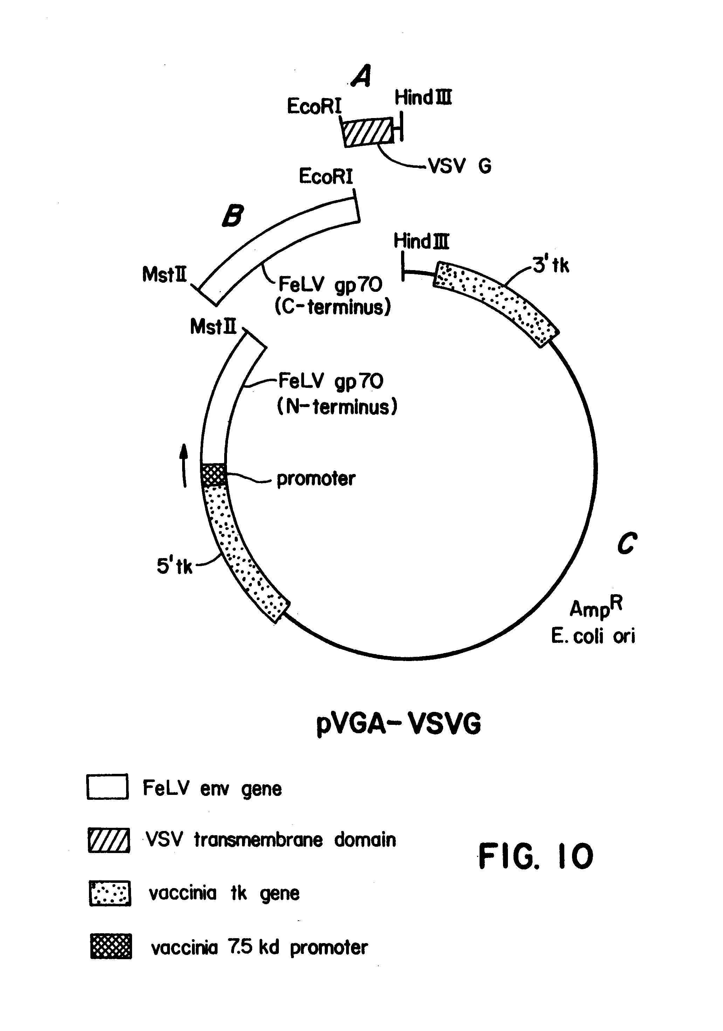

The nucleic acid sequences encoding such transmembrane protein regions can be inserted within the HgiAI site of sequences encoding for the gp70 protein of subgroup A or B FeLV. The HgiAI site is shown in Figures 2 and 10. The HgiAI site, near the C-terminus of the gp70 gene was used in the construction of the bacterial plasmid ptGAΔHgiAI, wherein the HgiAI site was converted to a more convenient EcoRI site. Insertion at such EcoRI site deletes the protein-cleavage site at which the FeLV gp85 precursor is normally processed to yield gp70 and p15E.

Samples of E. Coli k-12, strain MM294-1, transformed with the plasmid ptGAΔHgiAI were deposited under the Budapest Treaty at the American Type Culture Collection (ATCC), 12301 Parklawn Drive, Rockville, Maryland on December 2, 1983 and assigned the number 39,533.

The nucleic acid manipulations to construct the fusion of these protein domains can be conveniently performed in small plasmids, such as, ptGAΔHgiAI and then switched into the env gene of pVGA or pVGA-A of Examples 1 and 2, respectively. An MstII site is a conserved site within the gp70 region of both subgroup A and subgroup B FeLV viruses. The switching procedure can be accomplished by switching a fragment, stretching from the MstII site of gp70 to any convenient distal site, from said convenient small plasmid to an insertion vector, such as pVGA, which contains the entire FeLV gp85 region, and from which a comparable fragment (MstII to appropriate distal site) has been excised. Example 4 exemplifies such a procedure wherein fragments A and B represent the fragment which is switched. (See also Figure 10.)

Once the cassette with the gp70 env gene and alternative gene encoding a transmembrane protein has been switched into the appropriate insertion vector, the recombinant vaccinia virus can be prepared according to methods analogous to those described below in Example 1(B).

The viruses into which the cassette is ultimately incorporated should be capable of high copy level replication in cat cells and permit expression of the heterologous FeLV envelope gene during replication in cat cells. Viruses that normally infect cats, such as feline herpesvirus (FHV), may be used. Other viruses such as vaccinia virus and other pox viruses may be used provided they meet the requirements stated herein.

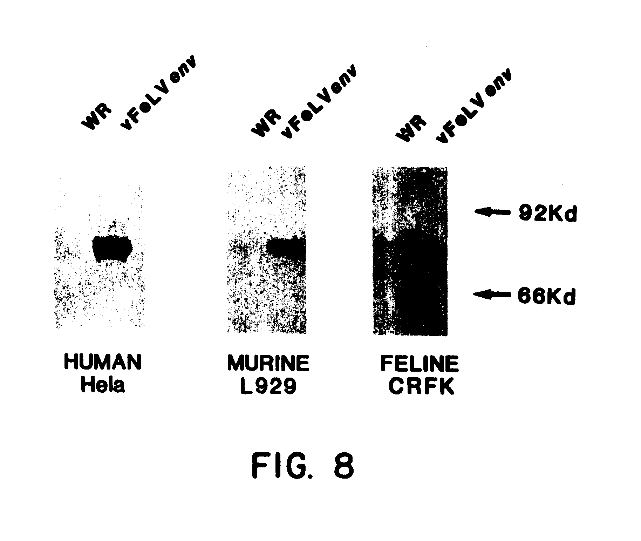

It is known that vaccinia virus is able to replicate in cats [Gaskell et al., Vet. Record, 112:164-170 (1983); Martland et al., Vet. Record. 112:171-172 (1983).] To quantitate the ability of vaccinia virus to replicate in cats, cells of feline, murine and human origin (CRFK or Fc9; L929; and Hela or 143B, respectively) were infected with the recombinant vaccinia virus of Example 1, and virus production and FeLV env protein accumulation were measured. [See Example 3(C) below and Figure 8.] The experiments indicated that replication of vaccinia virus strain WR, and the derived recombinant virus, is somewhat restricted in cells of feline and murine origin. Such decrease in virus production can be on the order of 1000-fold. Concomitant with such a decrease in virus production is a reduction in the amount of FeLV gp70 protein produced in vitro, and probably, in vivo.

In order to increase the level of production of FeLV gp70 immunogen, in vivo, it is preferable to use a virus vector, which has enhanced replication in cat cells. Such virus vectors with enhanced replication in cat cells are termed herein as feline-adapted vaccine virus vectors or host-range variants wherein cats are the host. It is preferred in each instance that the virus vectors employed in this invention be feline-adapted. Feline herpesvirus which is a preferred virus for use in this invention is an example of a naturally occurring feline-adapted virus.

To isolate host-range variants, which are not feline-adapted in their native state, the parental virus vector, for example, the vaccinia virus vector WR strain, or a derived recombinant virus, for example, the β-galactosidase marked virus (blue marked virus: vSC8 or vSC9) of Chakrabarti et al., [Mol. Cell. Biol. 5(12):3403-3490 (Dec. 1985)], is repeatedly passaged in feline cells under conditions which favor the emergence of host-range variants. McGuire et al. fully describe such procedures in their article entitled "Conditions Favouring the Emergence of Host Range Variants During Passage of a Poxvirus" [J. Gen. Virol., 19:301-310 (1973)]. Host-variants of the WR vaccinia virus have been produced which replicate with high efficiency in mouse cells and rabbit cells [Dunlap et al., J. Immunol., 100(6):1335-1339 (1968); and Gangemi et al., Virol., 73:165-172 (1976), respectively].

The heterologous FeLV envelope gene is incorporated into the virus by co-transfection of viral DNA and the insertion vector in mammalian cells. Depending on the particular virus involved, the cells may have to be infected with the virus to achieve productive infection. Various species of mammalian cells, including human cells, may be used with vaccinia virus; however, only cat cells may be used in this process with feline herpesvirus. Co-transfection allows for heterologous recombination between the nonessential region (e.g.,) TK sequences) and the viral DNA and the insertion vector, resulting in the insertion of the cassette into the viral genome. When the viral TK gene is used as the nonessential region, the gene is interrupted by the insert and recombinant viruses can be selected base on their TK-minus phenotype or by screening for the blue to white change according to the procedures of Chakrabarti et al, supra, wherein an expressible E. coli β-galactosidase (β-gal) gene is employed as a marker. Alternatively, recombinants may be identified by detecting the heterologous gene via DNA-DNA hybridization, or by detecting the presence of FeLV envelope protein produced during viral replication in the cells. After identification of the recombinants, pure recombinant virus may be isolated by conventional techniques and used to infect susceptible host cells to obtain large stocks of recombinant virus. The virus may be isolated from the infected cells and formulated with injectable vehicles for administration to cats by a variety of routes. The dose and dosage regimen used in the vaccination may vary depending upon the age and weight of the cat.

The following examples are intended to further illustrate the invention. They are not intended to limit the invention in any manner. The following materials and methods were employed in the examples set out below:

Materials and Methods

Cell Lines and Viruses. Human RD cells producing GA-FeLV virus [Mullins et al., J. Virol., 38:688-703 (1981)] were obtained from N. Davidson (Caltech). Canine CF-2 cells, expressing the molecularly cloned GA-FeLV provirus [Mullins et al., id], were provided by J. Elder (Research Institute of Scripps Clinic). The thymidine kinase-deficient human cell line, 143B [Weir et al., PNAS (USA), 79:1210-1214 (1982)] was from K. Huebner (The Wistar Institute) and murine L-929 cells were from L. Old (Memorial Sloan Kettering Cancer Institute). Human HeLa and feline CRFK [Crandell et al., In Vitro, 9:176-185 (1973)] cells were obtained from the American Type Culture Collection. The wild-type WR strain of vaccinia virus was obtained from B. Moss (NIH).

All cell lines, with the exception of CRFK, were grown in Dulbecco's Medium Eagle (DME; Irvine Scientific) supplemented with 10% fetal bovine serum (FBS) and Gentamycin (50 ug/ml; Schering). CRFK cells were grown in McCoy's 5A (Modified) medium (Gibco) supplemented as above.

Vaccinia virus was propagated by using standard techniques. Cell-associated vaccinia virus was isolated and purified by sucrose gradient centrifugation as described by Joklik, Virol., 18:9-18 (1962); viral DNA was prepared from purified virions as described by Nakano et al. [PNAS (USA), 79:1593-1596 (1982)]. For quantitation or isolation of vaccinia virus plaques, inoculated cell monolayers (Hela or 143B) were overlayed with medium containing 0.6% agarose, 2% FBS, and Gentamycin at 50 μg/ml. The

agarose overlay was additionally made 25 μg/ml in bromodeoxyuridine (BUdR) for the isolation of tk- virus plaques. FeLV virus from persistently infected cells was partially purified from culture supernatants by using methods described by Frankel et al., J. Virol., 18:481-490 (1976).

Specific Reagents. The vaccinia virus insertion vector, pGS20 [Mackett et al., J. Virol., 49:857-864 (1984)], was provided by G. Smith and B. Moss (NIH). This plasmid serves as a vector to permit the insertion and expression of foreign genes within the genome of vaccinia virus. A sample of E. coli HB101 transformed with pGS20 was deposited on November 30, 1982, at the American Type Culture Collection (ATCC), 12301 Parklawn Drive, Rockville, Maryland 20852 and has been given ATCC No. 39,249. Said deposit is referred to in International Publication Number WO 84/02077 (published 7 June 1984; based upon International Application Number PCT/US83/01863).

The molecularly cloned GA-FeLV-B provirus [λHF60] (Mullins et al., id) was provided by A. Roach and N. Davidson (Caltech) in the form of the pKC7-derived plasmid pKHR-1. This plasmid is infectious in transfection assays. The plasmid was pared down, to contain only the FeLV env gene and 3' LTR, by intramolecular recircularization following digestion with PvuII. This smaller plasmid, pGApv, provided the FeLV env gene sequence used in these studies.

The FeLV gp70-specific monoclonal antibody, clone 25.5, has been previously described [Lutz et al., Vet. Immunol. and Immunopath. 2:425-440 (1981)]. This IgGl antibody neutralizes FeLV infectivity and recognizes a defined determinant within FeLV gp70- [Nunberg et al., PNAS (USA), 81:3675-3679 (1984)]. Horseradish peroxidase (HRP) conjugates of the antibody, for enzyme-linked immunosorbent assays (ELISA), were prepared as described [Lutz et al., J. Immunol. Methods, 56:209-220 (1983)]. Another virus-neutralizing monoclonal antibody (C11D8) [Grant et al., J. Immunol., 131:3042-3048 (1983)] that recognizes the same epitope (unpublished) was provided by C. Grant (Pacific Northwest Research Foundation); this IgG2 antibody was used in experiments requiring antibody binding to Staphylococcus Protein A.

Recombinant DNA Methods. The recombinant DNA procedures used were as described by Maniatis et al. [Molecular Cloning: A Laboratory Manual, Cold Spring Harbor (1982)]. Nucleic acid enzymes were obtained from New England Biolabs or Bethesda Research Laboratories and were used as described by the manufacturer.

Recombinant Vaccinia Virus Methods. The general procedures used to derive recombinant vaccinia viruses have been described [Mackett et al., id.; Moss et al., Gene Amp. and Anal., 3:201-213 (1983)]. Vaccinia virus-infected 143B cells were transfected with vaccinia virus DNA and the pGS20-derived insertion vector containing the FeLV env gene (pVGA), and progeny virus were grown in the presence of BUdR (25 μg/ml) to select for recombinant (tk-) genomes. BUdR-resistant viruses were plaque-purified and screened for insertion and expression of the FeLV env gene as follows: Monolayers of Hela cells were infected with candidate viral isolates and harvested 2 days later by lysis in 0.1 M Na$_2$CO$_3$, pH 9 (ELISA coating buffer). A portion of the lysate was used directly in an ELISA assay to detect FeLV gp70. The lysate was used to coat microtiter wells, and FeLV env protein was detected using HRP-conjugated monoclonal antibody 25.5 and 2,2'-azino-di (3-ethylbenzthiazoline sulfonic acid) (ABTS;Sigma) substrate [Lutz et al., Vet. Immunol., 2:425-440 (1981)]. The remainder of the lysate was used to isolate high molecular weight DNA for dot hybridization analysis. The lysate was made to 0.05 M Tris (pH 7.0), 0.01 M EDTA, 0.5% SDS and was treated with proteinase K (50 μg/ml; Sigma) as described by Gross-Bellard et al., Eur. J. Biochem, 36:32-38 (1973). Following extraction with phenol and precipitation with ethanol, the DNA was redissolved in 0.1 M NaOH, 0.05 M EDTA for dot hybridization analysis as described by Kafatos et al., Nuc. Acids Res., 7: 1541-1552 (1979).

Immunological Analysis of FeLV env Protein Expression. Proteins were analyzed by SDS-polyacrylamide gel electrophoresis [Laemmli, Nature (London), 227:680-685 (1970)]. In some experiments, crude preparations of membrane proteins were isolated from virus-infected cells: Cells were swollen in 9 volumes cold hypotonic buffer (0.01M Tris, pH 7.4, 0.01 M NaCl, 0.001 M MgCl$_2$) and disrupted by Dounce homogenization. Nuclei were removed by low-speed centrifugation (1000xg, 10 min) and the membrane fraction was subsequently isolated by high-speed centrifugation at 29,000xg for 60 min. Protein content was determined by the method of Lowry, J. Biol. Chem., 193:265-275 (1951).

Immunoblot analysis was performed on proteins resolved by polyacrylamide gel electrophoresis and transferred electrophoretically to nitrocellulose (BA-85; Schleicher and Schuell) [Burnette, Anal. Biochem., 112:195-203 (1981); Towbin et al., PNAS (USA), 76:4350-4354 (1979)]. Blots were treated and incubated with antibody as previously described [Johnson et al., Gene Anal. Techn., 1:3-8 (1984); Nunberg et al., id.], and antibody-antigen complex was visualized using a HRP-conjugated second antibody (rabbit anti-mouse; Accurate Chemicals) and 3,3'-diaminobenzidine (Sigma) as the enzyme substrate [De Blas et al., Anal. Biochem., 133:214-219 (1983)].

Live cell indirect immunofluorescence experiments [Manger et al., Cell, 39:327-337 (1984)] were performed to localize FeLV env protein on the surface of vaccinia virus infected cells. Infected cell monolayers, grown in tissue culture chamber slides (Lab-Tek), were washed gently with phosphate-buffered saline (PBS) prior to incubation with monoclonal antibody 25.5 in PBS containing 4% bovine serum albumin (BSA). Following additional careful washing, immune complexes were visualized by reaction with a fluorescein-conjugated second antibody (rabbit anti-mouse IgG; Cappell Laboratories). Parallel monolayers were fixed in cold (-20°C) acetone prior to incubation with 25.5 antibody in order to detect total cellular FeLV env antigen. Slides were examined and photographed by ultraviolet epifluorescence microscopy.

Immunoprecipitation experiments were performed to further differentiate between FeLV env protein localized intracellularly or on the surface of infected cells. Infected cells were metabolically labelled during 19 hr of growth in methionine-free DME medium containing [$^{35}$S]-methionine (63 uCi/ml; New England Nuclear). In vaccinia virus infection, cells were labelled following a 2 hr inoculation period. Monolayers were washed with PBS and incubated with C11D8 monoclonal antibody (in PBS containing 1 mg/ml of BSA) in order to obtain reaction with FeLV env antigen on the surface of cells. The monolayers were then extensively washed prior to lysis with RIPA buffer containing 1% NP-40, 1% sodium deoxycholate, 0.1% SDS, 0.01 M methionine, 0.005 M EDTA, 0.005 M 2-mercaptoethanol, 1 mg/ml BSA, and 100 U/ml aprotinin (Boehringer-Mannheim) in PBS. Immune-complexes were precipitated using Staphylococcus Protein A-Sepharose (Sigma) and washed. Parallel immunoprecipitations were performed from cultures that had been lysed prior to incubation with antibody. Immunoprecipitated proteins were resolved by polyacrylamide gel electrophoresis and visualized by fluorography.

Example 1

This example describes the preparation of a live, recombinant vaccinia virus that carries the FeLV envelope gene of the Gardner-Arnstein strain of FeLV subgroup B.

A. Construction of the Insertion Vector

1. General Procedures

Procedures used in the isolation, restriction, and ligation of plasmid DNA and in the transformation of suitable bacterial hosts are standard procedures in the field of recombinant DNA, and may be found described in Maniatis et al., id.

All enzymes used in the construction of plasmid DNA vectors were obtained from either New England Biolabs or Bethesda Research Laboratories and were used according to supplier's specifications.

Unless otherwise noted, restriction digestions were to completion, utilizing 5-10 units enzyme per μg DNA, for 60 min at 37°C. Some digestions were purposely incomplete, and these generally utilized 0.05-0.5 units enzyme/μg DNA for 10-60 min. at 37°C, as determined experimentally.

Ligation reactions utilized T4 DNA ligase in a buffer composed of 66mM Tris-HCl, pH 7.6, 6.6mM MgCl₂, 10mM dithiotreitol (DTT), containing either 0.1mM ATP and 0.01-0.05 Weiss units ligase/20μl reaction volume for the ligation of sticky ends, or 1.0 mM ATP and 1.0-5.0 Weiss units enzyme/10μl reaction volume for the ligation of blunt ends. Reactions were carried out at 14°C for 6-12 hr. Reactions to produce intermolecular ligations contained 10-150μg vector DNA for sticky ends, and 100-200μg DNA for blunt ends. Insert DNA was maintained at a 1 to 20-fold excess to vector DNA.

Reactions to produce intramolecular ligations contained 10-20μg vector DNA.

2. Derivation of the pGS20-Derived Vector

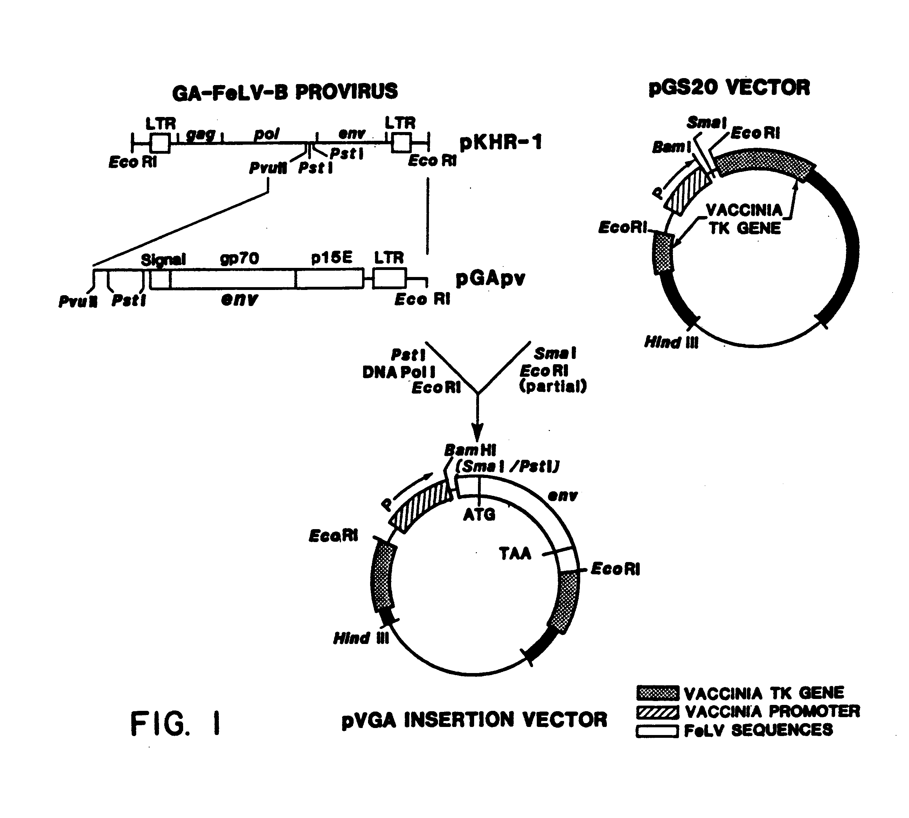

The plasmid vector utilized to mediate homologous recombination and insertion of the FeLV env gene into the vaccinia virus genome was derived from the previously described plasmid pGS20 [Mackett et al., J. Virol., 49:857-864 (1984)]. The pGS20 plasmid contains the vaccinia virus thymidine kinase (tk) gene interrupted by and inactivated by the insertion therein of the promoter region of the vaccinia virus gene encoding an early 7.5 kD protein [Venkatesan et al., Cell, 25:805-813 (1981)]. A convenient polylinker sequence has been inserted downstream from the transcription initiation site of the 7.5 kD gene in order to facilitate molecular cloning of heterologous sequences at this location (Figure 1).

This plasmid vector was digested to completion with BamHl. Following digestion and DNA isolation, the linear plasmid was incubated with Pol I (Klenow fragment) in the presence of 10 μM of each of the four deoxynucleotide triphosphates to form blunt ends. The BamHl-digested plasmid was then partially digested with EcoRI, under conditions designed to result in digestion at, on average, one of two sites. The DNA was purified and used as the vector for insertion of the FeLV envelope gene.

3. Derivation of the FeLV Envelope Gene and Insertion into the pGS20-Derived Vector

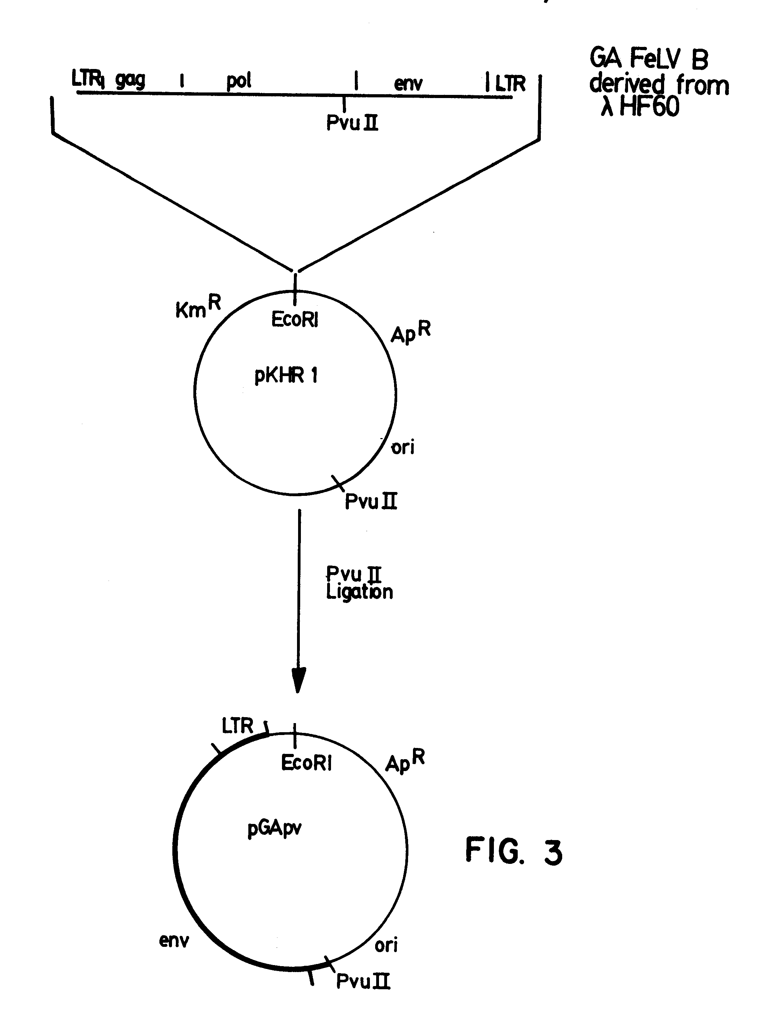

The plasmid pGApv was used as the source of the entire FeLV envelope gene sequence for subcloning into the above-described vector. pGApv was derived from pKHR-1 (A. Roach and N. Davidson), a plasmid containing the molecularly cloned provirus of the GA strain of FeLV-B (Figure 2) [lambda HF60, Mullins et al, J. Virol. (1981) 38:668], subcloned into the unique EcoRI site of the plasmid pKC7 (Rao and Rogers, Gene (1979) 7:79). pKHR-1 was digested with Pvull and religated under conditions favoring intramolecular reactions. This DNA was used to transform E. coli strain MM294 to ampicillin resistance. Cells harboring the 4.8 kb plasmid pGApv containing the entire envelope gene, but lacking most of the FeLV gag and pol genes and the bacterial kanamycin resistance gene, were isolated (Figure 3).

The FeLV envelope gene insert was prepared for subcloning by digestion of pGApv with Pstl. The DNA was incubated with Pol I (Klenow fragment) to generate blunt ends, isolated, and then digested with EcoRI (Figure 1).

The previously prepared BamHl/EcoRI (partially)-digested vector, pGS20, and the insert DNA were ligated as follows (Figure 1): the DNAs were mixed at a concentration of 150 μg/ml each with T4 DNA ligase under sticky-end conditions as described above. The mixture was incubated at 14°C for 6 hr., and additional enzyme and ATP were added to give previously defined blunt-end conditions, and incubation was continued overnight. The resulting DNA was used to transform E. coli strain MM294 to ampicillin resistance.

## 4. Identification of the Correct pGS20-FeLV Envelope Hybrid Insertion Vector, pVGA

Ampicillin resistant colonies harboring plasmids containing the FeLV envelope gene were located by the colony hybridization procedure (Maniatis et al., supra, p. 312-328). Nitrocellulose filter discs were laid on plates containing 500-800 ampicillin resistant colonies (from above) and marked for orientation. The discs were transferred, colony side up, to Whatman 3 MM paper saturated with 0.5 M NaOH, 1.5 M NaCl, and incubated for 5 min to solubilize the cells. The discs were neutralized by transfer to paper saturated with 0.5 M Tris-HCl, pH 7.4, 1.5 M NaCl, and incubated 5 min. The filters were washed in 0.3 M NaCl, 0.03 Na citrate (2x SSC), and 0.2% sodium dodecyl sulfate (SDS) three times for 5 min each, followed by washing three times, 5 min each, in 2x SSC alone. The filters were blotted dry and baked in vacuo at 80°C for 2 hr. The filter discs were probed by hybridization to $^{32}$p-labeled nick-translated FeLV envelope gene probe. The envelope gene sequences were derived from the 2.8 kb fragment generated by digestion of the FeLV provirus (contained in pKHR-1) with Xhol and EcoRl. This Xhol/EcoRl fragment was subcloned into the bacteriophage M13mp8, which had been digested with EcoRl and SalI. The entire M13mp8 recombinant was used in nick translation. Hybridization was as follows. Filters were incubated overnight at 42°C in 4 ml/filter of a prehybridization solution made up of 50% formamide, 5x SSC (1x = 0.15 M NaCl, 0.015 M Na citrate) 0.05x PPi (1x = 0.5 M Na$_2$HPO$_4$, 0.5 M NaH$_2$PO$_4$, 0.05 M Na$_4$P$_2$O$_7$), 0.1% SDS, 0.1% each Ficoll 400, bovine serum albumin, and polyvinylpyrrolidone, and 200 μg/ml denatured salmon sperm DNA. The filters were probed with 10$^6$ cpm/filter nick-translated FeLV env sequences (specific activity 1-5 x 10$^8$ cpm/μg) overnight at 42°C in 4 ml/filter of the same prehybridization solution. After hybridization, unhybridized probe was removed by washing 4 times for 15 min at 25°C in 2x SSC, 0.1% SDS, followed by 4 washes at 60°C in 0.1x SSC, 0.1% SDS. The filters were blotted dry and hydbridization visualized by autoradiography.

Colonies which contained the FeLV envelope gene sequences were isolated and grown and the plasmids were isolated by the rapid alkaline lysis procedure. The presence of the correct insertion at the desired EcoRl site was confirmed by digestion of the plasmids with BamHI and EcoRl, to yield a diagnostic 3 kb insert fragment.

## B. Construction of Recombinant Vaccinia Virus Containing the FeLV Envelope Gene

### 1. Cells and Viruses

The WR strain of vaccinia virus (obtained from B. Moss, NIH) was grown in HeLa Cells, and virus purified from cytoplasmic extracts by sucrose gradient centrifugation. Viral DNA was prepared as described by Nakano et al, PNAS (1982) 79:1592, with slight modifications. Briefly, purified virions at a concentration of 16.7 A260/ml were lysed in 0.01 M Tris-HCl, pH 7.8, 0.01 M EDTA, 0.1 M NaCl, 0.5% SDS, 0.05 M 2-mercaptoethanol. Protein was digested by incubation at 37°C for two hr in the presence of 50 μg/ml Proteinase K. The DNA was extracted by the addition of an equal volume of phenol, saturated with 0.001 M Tris-HCl, pH 7.8, 0.001 M EDTA, 0.1 M NaCl, and the aqueous phase reextracted until the interface was clean. The DNA solution was dialyzed overnight at 4°C against two changes of 0.01 M Tris-HCl, pH 7.8, 0.01 M EDTA. NaCl was added to 0.25 M and the DNA was precipitated by addition of 2.5 volumes -20°C 100% ethanol. The precipitated DNA was spooled from the solution on a pasteur pipette, transferred to 80% ethanol (-20°C), pelleted by centrifugation at 12,000x g for 5 min, and dried. The vaccinia DNA was dissolved in 0.01 M Tris-HCl, pH 7.4, 0.001 M EDTA, and the concentration determined by A260.

HeLa cells and human 143B TK-minus (obtained from K. Huebner, Wistar Institute) were grown in Dulbecco's Medium Eagle (DME) supplemented with 10% fetal bovine serum. For selection of TK-minus variants of vaccinia virus, the 143B cells were grown in medium containing, in addition, 25μg/ml bromodeoxyuridine (BUdR).

Plaque assays were performed by inoculating confluent monolayers of 143B cells with dilutions of virus stocks for 3 hr at 37°C with intermittent rocking. The inocula were removed, the cells washed once, and the monolayer overlayed with DME containing 2% fetal bovine serum and 0.6% agarose. After 48-72 hr incubation at 37°C, plaques were visualized by incubating the culture for 3 hr with 0.005% neutral red in phosphate-buffered saline. For isolation well-isolated plaques were picked with a sterile glass pasteur pipette, transferred to 1 ml medium, and vigorously pipetted to release the virus from the agarose plug. This suspension was used to infect duplicate 143B cell monolayers in 16 mm diameter wells.

### 2. Transfection of Vaccinia Virus-Infected Cells With Insertion Vector, pVGA, and Vaccinia DNA to Permit In Vivo Homologous Recombination and Isolation of TK-Minus Mutant Viruses

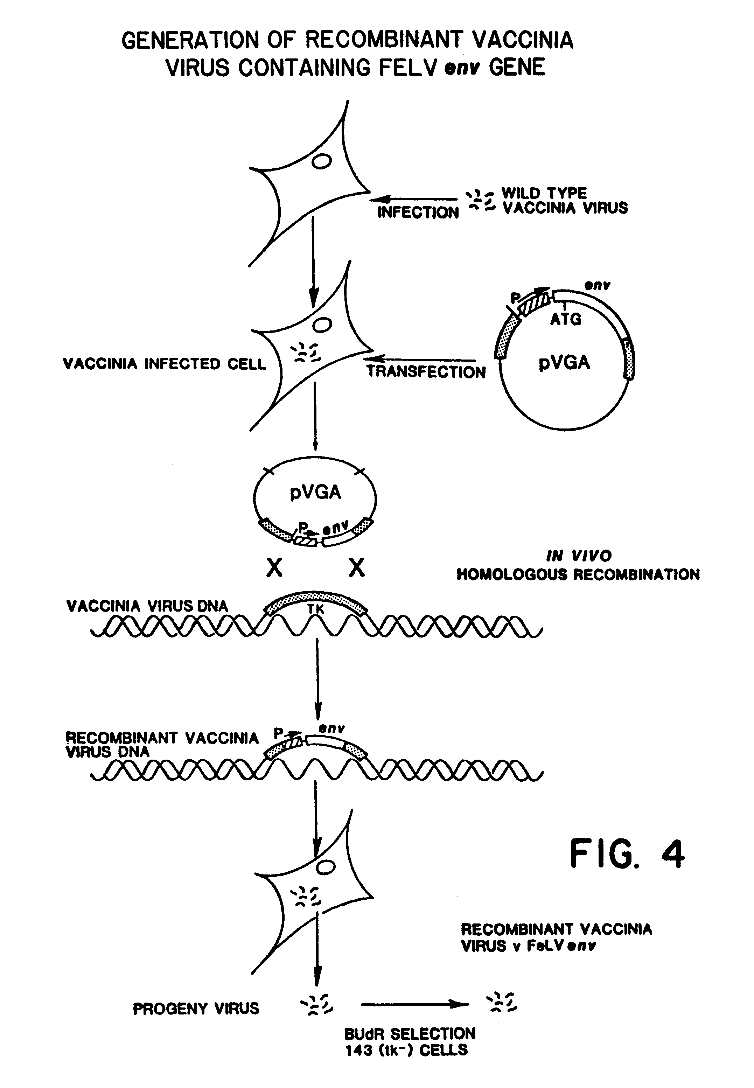

Recombination of the FeLV envelope gene into the TK gene of wild-type vaccinia virus was accomplished using the marker rescue techniques as described by Mackett et al, PNAS (1982) 79:7415 (Figure 4). Briefly, 60 mm tissue culture dishes containing confluent monolayers of 143B TK-minus cells were infected with the WR strain of vaccinia virus at a multiplicity of infection of 0.05 plaque-forming units (pfu) per cell. Two hours post infection, the vaccinia virus-infected cells were transfected with a mixture of insertion vector and vaccinia viral DNA by the calcium phosphate co-precipitation procedure. The transfection was performed as follows. One μg each of vaccinia viral DNA and pVGA DNA were mixed with 20 μg carrier salmon sperm DNA in a volume of 50 μl. The solution was brought to 0.5 ml with 0.25 M CaCl$_2$ and slowly added, with constant agitation, to 0.5 ml 2x HBS (280 mM NaCl, 1 mM KCl, 1.5 mM Na$_2$HPO$_4$, 5.5. mM dextrose, 25 mM Hepes, pH 7.05), and the precipitate was allowed to form 30 min at room temperature. The DNA precipitate was added to the vaccinia virus-infected cell monolayer and incubated for 30 min at 37°C. Five ml growth medium was added, and the incubation continued for 4-6 hr at 37°C. The DNA solution was removed, the monolayer washed once, and 3 ml of 15% glycerol in DME was added and incubated for 3 min at room temperature. The glycerol solution was removed, the cells washed three times, and 5 ml growth medium added. Forty-eight hours post infection, the cultures, showing the expected extensive cytopathic effect, were harvested by shaking the cells off the dish, pelleting ten minutes at 1000x g, and resuspending in 1 ml medium.

The virus stock isolated from the transfection of vaccinia-infected cells comprised a mixture of TK-minus virus in the presence of a vast excess of wild-type vaccinia virus. In order to isolate TK-minus variants, some of which are recombinants, the stock was plaqued, as described above, on TK-minus human 143B cells in the presence of 25 μg/ml BUdR. Under these conditions, only those viruses lacking TK activity were able to form plaques. Individual well-isolated plaques were picked through the agarose overlay, suspended in medium, and transferred to 16 mm diameter wells containing monolayers of 143B cells. These virus clones were grown in the presence of BUdR, and the infected cells were used as the source of material for screening for the presence and expression of the FeLV envelope gene by DNA hybridization and enzyme-linked immunosorbent assay (ELISA), as described below.

### 3. Identification of recombinant Vaccinia Virus Expressing the FeLV Envelope Gene

The TK-minus virus isolated by plaque purification in the presence of BUdR is a mixture of spontaneous TK-minus mutants and virus rendered TK-minus by insertion of the FeLV envelope gene into the viral TK gene by homologous recombination. Two methods were employed to screen for FeLV-containing vaccinia virus. Cells infected with plaque-purified TK-minus vaccinia were assayed for the presence of the FeLV sequence by DNA hybridization. Briefly, high molecular weight DNA was prepared by a modification of the method of Gross-Bellard et al., Eur. J. Biochem. (1973) 36:32. Infected cells were solubilized in 0.1 M Na2Co3, pH 9.0. 150 μl of lysate was added to 250 μl of 0.01 M Tris-HCl, pH 7.0, 0.01 M EDTA, 0.5% SDS and 50 μg/ml Proteinase K. The solution was incubated 60 min at 37°C and then extracted two times with an equal volume of phenol. The DNA was precipitated from solution by addition of 2.5 volumes 100% ethanol. The DNA was pelleted 5 min at 12,000x g, dried, and dissolved in 100 μl 0.1 M NaOH, 0.005 M EDTA. DNA aliquots, 50 μl, were bound to nitrocellulose filters by the procedure of Kafatos et al., Nuc. Acids Res. (1979) 7:1541. Filters were incubated overnight at 37°C in the prehybridization solution described above, and incubated 24 hr at 37°C in the presence of 10^6 cpm/filter [32]p-labeled nick-translated M13mp8 DNA containing the XhoIEcoRI FeLV envelope gene fragment described above. Four washes in 2 x SSC, 0.1% SDS at 25°C were followed by two 60 min washes in 0.1x SSC, 0.1% SDS. Filters were used to expose x-ray film to identify samples containing FeLV envelope gene sequences.

Virus clones which were positive by DNA hybridization were then assayed by ELISA for the expression of the envelope gene. Infected cells were removed from the 16 mm diameter well by vigorous pipetting and pelleted at 12,000x g for 10 min. The pellet was resuspended and solubilized in 200 μg of ELISA coating buffer (0.1 M Na2CO3, 0.02% NaN3, pH 9.6). 100μl aliquots of serial two-fold dilutions in coating buffer, beginning at 1:4, were added to wells of microtiter plates. The proteins were bound to the plastic wells by incubating at 37°C for three hours. The plates were then washed three times with wash buffer (0.15 M NaCl, 0.05% Tween-20), and excess liquid removed. FeLV envelope gene product in the wells was detected using a monoclonal antibody, clone 25.5, which recognizes a determinant on the FeLV gp70 protein (Lutz et al, Vet. Immuno. and Immunopath. (1981) 2:425, Nunberg et al, PNAS (1984) 81:3675). The IgG was purified from ascites fluid (Lutz et al., J. Immuno, Methods (1983) 56:209) and conjugated with horseradish peroxidase as the reporter molecule. The conjugate was diluted 1000-fold in Buffer 3 (0.15 M NaCl, 0.001 M EDTA, 0.05 M Tris-HCl, pH 7.4, 0.05% Tween-20, 0.1% bovine serum albumin). 100 μl of antibody solution was added to each well, and antibody was allowed to react with antigen for 60 min at 37°C. The unbound antibody was removed by washing the wells three times with wash buffer. Bound antibody was detected with 100 μl substrate solution (12.5 μM 2,2-azino-di-(3-ethylbenzthiazoline sulfonic acid) (ABTS), 0.0005% H2O2, 0.05 M citric acid, pH 4.0) per well. The green color was allowed to develop for 20 min at room temperature. The reaction was stopped by the addition of 100 μl 0.2 M HF, and the absorbance at 405 nm determined. The reactivity of the vaccinia virus-infected cells was compared to that of human cells productively infected with authentic FeLV. Clones shown strong expression of envelope gene product were plaque purified a second time in the presence of BUdR, and several recombinant vaccinia virus clones expressing FeLV envelope protein were isolated and designated vFeLVenv.

Said recombinant vaccinia virus expressing the feline leukemia virus envelope gene (vFeLVenv) was deposited on October 1, 1985 at the American Type Culture Collection (ATCC) under the ATCC Accession No. VR2215.

Example 2

pVGA-A - Construction of Insertion Vector
Containing the FeLV env Gene of FeLV Subgroup A

This example indicates that a live, recombinant vaccinia virus that carries the FeLV envelope gene of FeLV subgroup A can be prepared essentially according to the procedures of Example 1(A) and (B). A molecularly cloned subgroup A virus [Stewart et al., J. Virol., Vol. 58(3):825-834 (June 1986)] is employed instead of the Gardner-Arnstein strain subgroup B virus.

A BalI to BalI fragment of the subgroup B env gene in pVGA spans the region that encodes from amino acid 1 or mature gp70 to a point within the conserved p15E protein. [Figure 2] Such Bal I to Ball sites are conserved sites in both subgroup A and subgroup B viruses (Stewart et al., supra.) Said Ball to Ball fragment from the subgroup B virus can be replaced by an identical fragment from the subgroup A virus by methods known to those of ordinary skill in the art to derive plasmid pVGA-A, which contains the gene encoding the entire gp70 and p15E proteins of subgroup A FeLV. Such construction would be essentially analogous to that of Example 1(A).

Employing pVGA-A, recombinant vaccinia virus can then be constructed by the same procedures as described in Example 1 (B), preferably using a feline-adapted vaccinia virus as a vector.

## Example 3

Characterization of FeLV Envelope Gene Expression by the Recombinant Vaccinia Virus vFeLVenv

A. Immunoblot Analysis of vFeLVenv-Encoded Expression of FeLV env Gene

The molecular weight of the FeLV envelope gene product expressed by the recombinant vaccinia virus of vFeLVenv was determined by immunologic (Western) analysis. HeLa cell monolayers were infected at an MOI of 1 pfu/cell with either the recombinant vFeLVenv or the WR strain of vaccinia virus. Twenty-four hours post infection, the cells were scraped from the culture vessel and pelleted at 800x g for 10 min. The pellets were dissolved in SDS-PAGE sample buffer (2% SDS, 0.1 M Tris-HCl, pH 7.0, 0.1 M DTT, 10% glycerol, 0.1% bromphenol blue), sonicated for 3 min, and heated to 100°C for 10 min.

Authentic FeLV envelope protein samples were obtained by preparation of crude membrane fractions of human RD cells productively infected with GA-FeLV B. Approximately $10^8$ cells were pelleted at 800x g for 10 min, washed once with phosphate-buffered saline, and resuspended in 9 volumes of RSB (0.01 M Tris-HCl, pH 7.4, 0.01 M NaCl, 0.001 M MgCl$_2$), and incubated 10 min at 4°C. Cells were lysed with 20 strokes of dounce homogenizer. The suspension was clarified at 1,600x g for 10 min, and the supernate was centrifuged at 29,000x g for 60 min. This crude membrane pellet was suspended in 0.01 M Tris-HCl, pH 7.4, 0.001 M EDTA, dissolved in SDS-PAGE sample buffer, and heated to 100°C for 10 min.

Proteins were separated electrophoretically on 10% acrylamide gel. After separation, the proteins were transferred to nitrocellulose in 0.05 M sodium acetate, pH 7.0, for 60 min at 0.1 V/cm$^2$. Following transfer, the filter was washed 30 min in 250 ml of 5% non-fat dry milk, 1% ovalbumin, 1 M glycine. The filter was washed 3x 5 min each in 250 ml wash buffer (0.01 Tris-HCl, pH 7.4, 0.15 M NaCl, 0.001 M EDTA, 0.1% SDS, 0.2% Triton X-100, 1% non-fat dry milk, 0.1% ovalbumin). FeLV envelope protein was detected by incubating the filter with monoclonal antibody clone 25.5. The ascites fluid was diluted 1:200 in wash buffer and incubated with the filter either for 3 hr at 25°C or overnight at 4°C. The unreacted antibody was removed by three washes for 5 min in 250 ml wash buffer. Rabbit anti-mouse IgG coupled with horseradish peroxidase was diluted in wash buffer according to manufacturer's (Accurate Chemicals Co.) specifications and incubated with the filter for 1 hr at 25°C. Unreacted antibody was removed by three 5-min 250-ml washes in wash buffer. Immunoreactive proteins were visualized by incubating the filter with enzyme substrate, 0.5 mg/ml diaminobenzidine, 0.03% H$_2$O$_2$, in 0.1 M Tris-HCl, pH 7.5.

As shown in Figure 5, the protein produced by the recombinant vaccinia virus-infected cells co-migrates with that made by cells infected with authentic FeLV. Immunoblot analysis of proteins expressed by vFeLVenv-infected Hela cells revealed a single band of immunoreactive material that migrated with an apparent molecular size of 83kD (Fig. 5). This protein was enriched in membranes prepared from these cells, which observation is in the line with the expected membrane association of the env gene product. A protein of identical molecular size was observed in membrane preparations of dog cells, productivity infected with authentic GA-FeLV virus (Fig. 5).

The apparent molecular size of the 83kD protein does not in itself distinguish between the two potential forms of the FeLV env gene product - the 'gp85' precursor protein or the processed, mature 'gp70' protein. To obtain a molecular size marker for mature 'gp70' of GA-FeLV, FeLV virions containing 'gp70' were partially purified from culture supernatants of GA-FeLV-infected CF/2 canine cells. Immunoblot analysis of virion protein (Fig. 5, last lane marked GA-FeLV virus) revealed a band that comigrated with those previously observed in membrane preparations of cells infected with either vFeLV env or authentic GA-FeLV. Thus, the env gene product that accumulates in these cells is the mature, processed form of the protein, gp70. This assignment has been confirmed by molecular size analysis of the protein product after treatment with anhydrous hydrogen fluoride [Mort et al., Anal. Biochem., 82:289-301 (1977)] to remove carbohydrate moieties (5lkD; data not shown). The apparent 83kD molecular size of the GA-FeLV 'gp70' glycoprotein likely reflects the large number of potential glycosylation sites present within this protein relative to other retroviral 'gp70' proteins [Elder et al., J. Virol., 46:871-880 (1983)].

In these analyses of steady-state protein levels, no accumulation of a higher molecular size 'gp85' precursor protein was observed. That proteolytic processing to generate mature gp70 occurs efficiently in cells infected with vFeLVenv demonstrates that cleavage of the gp85 precursor does not require the presence of other FeLV-encoded proteins and is independent of the process of FeLV virus assembly and budding.

## B. Localization of the Expressed Envelope Gene Product to the Infected Cell Surface

### (1) Indirect Immunofluorescent Experiments

To further evaluate the nature of the expression of the FeLV envelope gene by the recombinant vaccinia virus, the cellular localization of the expressed protein was determined, using a live cell immunofluorescent assay similar to that described by Manger et al., Cell (1984) 39:327-337.

Tissue culture chamber slides (Lab-Tek), containing monolayers of 143B cells at 60-75% confluency were infected with the WR strain of vaccinia virus or with the recombinant vaccine vFeLVenv at an MOI of 10-20 pfu/cell. At 3 hr, 6 hr, and 24 hr post infection, cells (fixed or unfixed, described below) were incubated with antibody to detect expression of the FeLV envelope gene product as follows. Cells were carefully washed 3 times with 4°C PBS. Cells were either unfixed, where only those antigens on the surface of the cells are accessible to antibody, or fixed, 15 min in -20°C acetone, where the internal antigens of the cells are also available to antibody. The cells (fixed or unfixed) were incubated for 60 min at 25°C with 1:200 dilution of monoclonal antibody clone 25.5 (described above) ascites fluid, in PBS plus 4% BSA. The cells were washed carefully 3x with PBS, and reacted for 60 min at 25°C with rabbit anti-mouse IgG conjugated with fluorescein isothiocyanate diluted 1:50 in PBS plus 4% BSA. The cells were again washed carefully 3x with PBS, and coverslips mounted with 50% glycerol in PBS. The cells were viewed by ultraviolet epifluorescence and photographed.

The results of these immunofluorescent analyses are seen in Figure 6. In panel B the cells are unfixed and only the surface is available. In panel A the cells are fixed, exposing the interior of the cells to antibody. The FeLV envelope gene product was clearly expressed in cells as early as 3 hr post infection. Surface fluorescence was visible after 6 hours. In addition, 24 hr post infection, unfixed cells (panel B) showed the characteristic intense cell surface staining, indicating the envelope gene product was located on the surface of the recombinant vaccinia vFeLVenv-infected cells. Thus, the results of this immunofluorescence assay indicated that the env gp70 protein is transported to, and accumulates on, the surface of cells infected with the recombinant vaccinia virus.

### (2) Immunoprecipitation Experiments

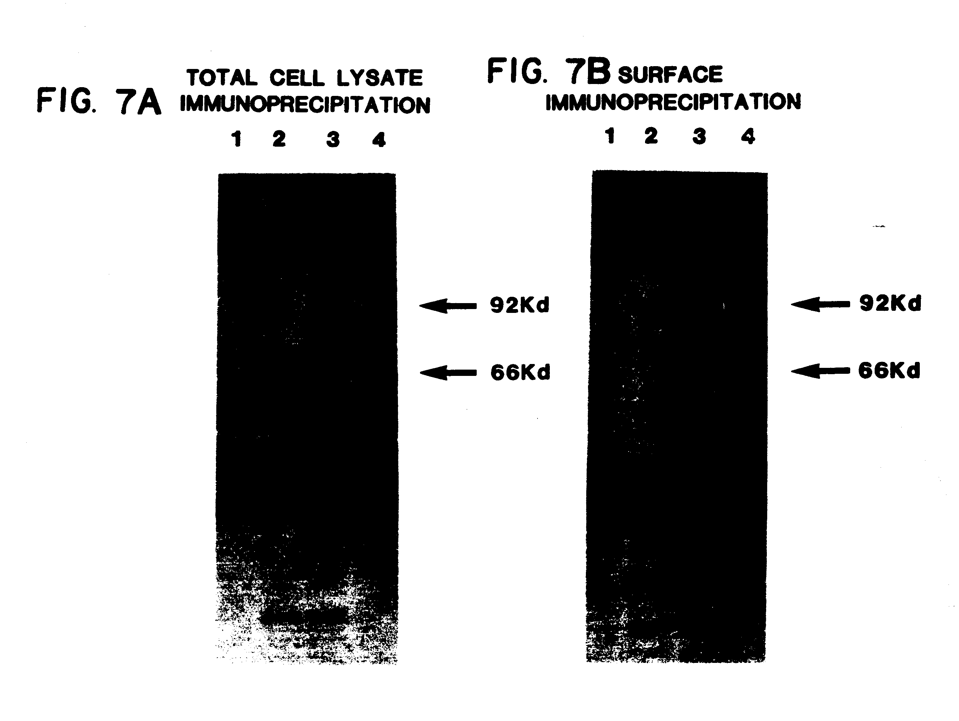

To quantitate these findings, and to compare the transport of the env gene product in vFeLVenv-infected cells with that in cells infected with FeLV, immunoprecipitation experiments were performed so as to distinguish between intracellular and surface expression of gp70 antigen. Cell monolayers were infected with vaccinia virus and metabolically labelled for 19 hr. The intact monolayer was then incubated with an anti-FeLV gp70 monoclonal antibody [C11D8 (Grant et al., J. Immunol., 131:3042-3048 (1983)] and washed extensively prior to detergent-lysis and immunoprecipitation by using Staph A protein; in this way, only surface gp70 antigen is precipitated. Total cellular gp70 antigen was immunoprecipitated from parallel cultures that were lysed before incubation with antibody. Figure 7 compares the results obtained from cells infected with the recombinant vaccinia virus vFeLVenv (lane 3 in both instances) with those obtained from cells persistently infected with authentic GA-FeLV (lane 4 in both instances).

As indicated in the Brief Description of the Drawings, supra, for Figure 8, $1 \times 10^7$ cells were not lysed prior to incubation with antibody (Surface Immunoprecipitation results) whereas $2.5 \times 10^6$ cells were lysed prior to incubation with antibody (Total Cell Lysate Immunoprecipitation results). Judging from the immunoprecipitation results in comparison with the cell numbers treated, it can be seen that the gp70 protein in vFeLVenv-infected cells appears to partition roughly equally between intracellular and cell-surface membranes, whereas in cells productively infected with FeLV, less that 10% of the gp70 protein accumulates on the cell surface. These results indicate that cells infected with the recombinant vaccinia virus accumulate on their surfaces substantially more gp70 than do cells infected with FeLV.

In summary, we find no qualitative differences in the synthesis, processing, and transport of the FeLV env protein when expressed in cells infected with the recombinant vaccinia virus, vFeLVenv, or in cells infected with FeLV. Proteolytic processing of the env gp85 precursor, to yield mature gp70 occurs rapidly during transit to the cell surface where it accumulates.

## C. Expression of vFeLVenv-encoded FeLV env Gene in Feline Cells

In the experiments described in this section, vaccinia virus replication and FeLV env gene expression was examined in cells of feline origin. From the veterinary literature [Gaskell et al., Vet. Record, 112:164-170 (1983); Martland et al., Vet. Record., 112:171-172 (1983)], and from unpublished safety studies performed by the instant inventors, it was known that vaccinia virus is able to replicate in cats. To quantitate these aspects, cells of feline, murine, and human origin (CRFK or Fc9; L929; and Hela or 143B, respectively) were infected with the recombinant vaccinia virus, and virus production and FeLV env protein accumulation were measured. Comparable cell monolayers were infected at a multiplicity of infection of one and cells were harvested at 48 hours post-infection. Cell-associated virus was quantitated in a plaque assay using Hela cells, and gp70 accumulation was determined immunologically from membrane preparations of infected cells.

These experiments confirmed that substantial vaccinia virus replication occurs in cells of feline origin. Virus yields obtained in feline cells were, however, substantially reduced from those obtained in human cells ($10^8$-$10^9$ pfu vs. $10^{10}$-$10^{12}$ pfu per $10^7$ cells, respectively). Virus replication in feline cells was further evidenced by the extensive, albeit slower, cytopathic effect observed in infected feline cell monolayers. A similar pattern of virus growth was observed in cells of murine origin; virus yields obtained in mouse L929 cells were generally comparable to those obtained in cat cells.

In all cell lines examined, virus yields were similar in cells infected with the wild-type WR virus or with the recombinant vFeLVenv virus (data not shown). Expression of the FeLV env gene product, thus, did not appear to affect the level of virus production. Indeed, to a first approximation, expression of FeLV gp70 protein in vFeLVenv-infected cells appeared to correlate with the observed levels of virus production. That is, all cell lines produced FeLV gp70 protein during infection; in keeping with the relative levels of virus production, accumulation of the gp70 protein in feline and murine cells was substantially reduced as compared to that in human cells (Fig. 8).

In comparing feline and murine cells, however, the direct relationship between virus yield and FeLV gp70 accumulation is ostensibly weakened. Although virus yields were comparable in infected murine and feline cells, murine cells were consistently found to accumulate higher levels of FeLV gp70 protein.

Example 4

Insertion Vector Containing FeLV gp70 and Sequence Encoding for the VSV G Protein Transmembrane Region

The construction of a preferred insertion vector, wherein the vesicular stomatitis virus (VSV) G transmembrane region replaces the p15E region of pVGA prepared according to the procedure of Example 1, is described in this example.

A. VSV G Fragment

The VSV G protein cDNA of pGF1 [Rose et al., J. Virol., 39:519-528 (1981)] is digested to completion with Taq I (nucleotide 1291) and N1alII (nucleotide 1591). The 300 bp fragment containing the transmembrane region and termination codon is then isolated.

Said 300 bp fragment is then molecularly cloned into pUC18 at the compatible AccI and SphI sites. The resulting plasmid, pUC8-VSVG, is then digested with EcoRI and HindIII to liberate the VSV G fragment with suitable polylinker-derived restriction sites at the temini (Section A as shown in Figure 10).

B. 560 BP Fragment

1. ptGAΔHgiAI

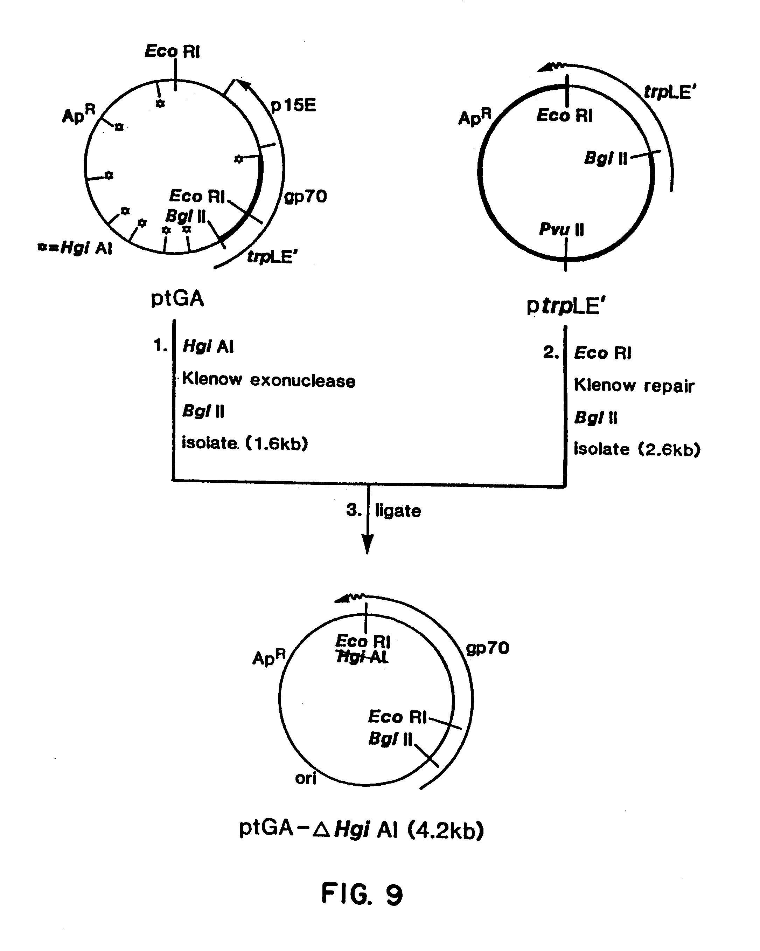

The plasmid ptGAΔHgiAI encodes a trpLE' fusion protein encoding all but the extreme C-terminus of FeLV gp70. The derivation of ptGAΔHgiAI is outlined in Figure 9.

The construction, source and derivation of plasmids ptGA and ptrpLE' are described in detail in PCT/US84/01963 (published 18 December 1985). Said PCT published patent application is in pertinent part herein incorporated by reference. Plasmid ptGA contains sequences of the entire FeLV gp 85 precursor protein fused to trp LE' as diagrammed in Figure 9.

The plasmid ptGA was digested with HgiAI (HgiAI:

1-5 units/µg, 30-60 min, 37°C) and the HgiAI 3' protruding ends were degraded, to generate blunt ends, using the 3'-5' exonuclease activity of the Klenow fragment of DNA polymerase I. This material was then digested with BgIII, and the 1.6 kb BgIII-blunt HgiAI fragment was isolated by polyacrylamide gel electrophoresis. The plasmid ptrpLE' was digested with EcoRI ends repaired with Klenow fragment. This material was then digested with BgIII, and the 2.6 kb vector fragment isolated by gel electrophoresis. The 1.6 kb BgIII-blunt HgiAI fragment and the 2.6 kb BgIII-repaired EcoRI vector fragment were combined and ligated in a two-step process (intermolecular sticky-end, plus intramolecular blunt-end). This material was used to transform E. coli to ampicillin resistance. Plasmids from resulting colonies were screened for regeneration of EcoRI and BgIII sites. The desired plasmid is indicated ptGAΔHgiAI in Figure 9.

2. Digestion of ptGAΔHgiAI to Yield 560bp Fragment

The plasmid ptGAΔHgiAI prepared according to the procedures outlined immediately above in 4(B)(1) is then digested with EcoRI (at the former HgiAI site near the C-terminus of FeLV gp70) and with MstII (within FeLV gp70). The 560 bp fragment cleaved by said digestion which contains approximately half of the C-terminal FeLV gp70 gene is then isolated (Section B as shown in Figure 10).

C. Large Fragment Containing Vaccinia tk Gene; E. Coli ori; AmpR; Vaccinia 7.5 KD Promoter and Portion of FeLV gp70

The plasmid pVGA (from Example 1) is then partially digested with MstII (within FeLV gp70) and partially digested with HindIII (within the human genomic sequences flanking the FeLV provirus in the original plasmid pKHR-1 and in pVGA derived therefrom). Such a partial digestion of pVGA, is carried out under conditions designed to result on the average, in only one each of the two MstII and two HindIII sites being cleaved. A large fragment (shown in Figure 10 as section C of pVGA-VSVG) is then isolated. Said fragment contains in a clockwise direction the HindIII site through the 3' half of the vaccinia virus TK gene from pGS20; and then through the entire pGS20 plasmid which includes an E. coli origin of replication and an ampicillin-resistance marker; and further through the 5' half of the pGS20 TK gene, the pGS20 7.5 KD promoter and finally to the MstII site in the middle of FeLV gp70.

D. pVGA-VSVG

The three fragments isolated from the preparations described immediately above in 4(A), 4(B) and 4(C) and designated respectively as sections A, B and C, are then mixed and ligated to obtain the desired plasmid containing the sequence encoding for the VSV G protein transmembrane region fused at the C-terminal end of FeLV gp70. Said sequence replaces the FeLV p15E region of pVGA.

Such plasmid designated pVGA-VSVG is illustrated in Figure 10. The recombinant vaccinia virus can then be prepared analogously to the methods described above in Example 1(B). The resulting fusion protein expressed through infection with said recombinant vaccinia virus would contain almost the entire FeLV gp70 env protein and the VSV G protein transmembrane region to ensure anchoring within the membrane.

Other nearby sites and analogous strategies directed around such sites can be employed to prepare conceptually equivalent fusions wherein the cassette comprises a gp70 region and a transmembrane region other then the FeLV p15E region.

Example 5

FHV

This example describes the use of feline herpesvirus (FHV) as an alternative to vaccinia virus in preparing live, recombinant virus vaccines for feline leukemia. FHV is a naturally occurring virus of the herpesvirus family. It infects cats, resulting in feline rhinotracheitis disease, a respiratory disease producing substantial morbidity and mortality. Domestic cats are routinely vaccinated against the feline rhinotracteitis virus using several commercially available killed virus or attenuated live virus vaccines. The current use of the attenuated live FHV vaccine permits the use of a live, recombinant FHV virus vaccine that includes the FeLV envelope gene which, aside from protecting against rhinotracheitis, also protects against FeLV as a result of the expression of the FeLV envelope gene. An FHV vaccine strain that is attenuated is preferred.

The methodology for constructing an FHV-based recombinant virus containing the FeLV envelope gene follows that of Example 1 and involves the following steps:
- molecular cloning of the FHV TK gene and flanking sequences,
- insertion within the TK gene of a recombinant DNA cassette containing the entire FeLV envelope gene or the FeLV gp70 gene and sequence encoding for a transmembrane region protein from an alternative source and a suitable upstream promoter [for example, such promoters would include the HSV I TK promoter, the HSV α-4 promoter, the FHV TK promoter, or a FHV capsid or major envelope glycoprotein promoter] to drive transcription,
- co-transfection of feline cells with plasmid constructed above and FHV genomic DNA to permit in vitro homologous recombination of FHV TK sequences and the introduction of the promoter-FeLV envelope gene cassette into the genome of FHV,
- isolation of progeny viruses from the transfection containing wild-type and recombinant viruses, and
- isolation of TK-minus viruses expressing the FeLV envelope gene product.

These steps are detailed below.

A. Molecular Cloning of FHV TK Gene

A recombinant DNA library containing all restriction fragments of the FHV genome, including the TK gene, is constructed as follows. FHV genomic DNA is isolated as described by Pignatti et al [Virology (1979) 93:260-264] and digested to completion by either of several infrequently cutting restriction endonucleases (e.g., EcoRI, BamHI, HindIII, etc.) and the resulting fragments are molecularly cloned into either plasmid or phage lambda vectors as described by Maniatis et al. (supra). Assuming the average restriction fragment from these digestions is 5 kb, approximately 20 different clones are required to include the entire FHV genome of approximately 100 kb. Several approaches are used to identify the specific molecular clone containing the TK gene.

One method is based on the ability of the molecularly cloned FHV TK gene to complement a TK deficiency in the host cell. This method requires isolation of a TK-minus feline cell line. A TK-minus feline cell line may be isolated from feline CRFK cells (Crandell et al., In Vitro (2974) 9:176-185) by selection in the presence of BUdR or by chemical mutagenesis (e.g., Caboche, Genetics (1974) 77:309-322; van Daalen Wetters and Coffino, Mol. Cell Biol. (1982) 2:1229-1237) of the CRFK cells prior to growth under selective conditions. Cell clones which are able to grow at the highest concentrations of BUdR are isolated. Lack of TK enzymatic activity is confirmed by assaying cell lysates for TK activity (Lee and Cheng, J. Biol. Chem. (1976) 251:2600-2604). Having obtained a TK-minus feline cell line, transfection experiments such as those described above are performed to identify molecularly cloned sequences capable of complementing the TK-minus phenotype in HAT selective medium.

An alternative method that does not require a TK-minus feline cell line follows the procedure developed by Kaplan and Ben-Porat, Virology (1961) 13:78-92, for isolating the PrV TK gene. Cells treated with the thymidine analog 5-fluorouracil (FU), followed by treatment with thymidine, are rendered irreversibly unable to utilized thymidine by either the de novo or salvage (TK) pathways. These cells are unable to divide but are able to support the replication of herpesviruses containing a functional TK gene.

Each of the approximately 20 distinct molecularly cloned FHV fragments is co-transfected, along with FHV DNA from an araT-resistant (TK-minus) FHV virus, into feline cells, and progeny virus is isolated. If the molecularly cloned fragment contains the TK gene, then some progeny viruses will contain a functional TK gene by virtue of having recombined with the transfected wild-type TK gene fragment. The progeny viruses are then used to infect cat cells which have been treated with FU plus thymidine. Only virus populations containing TK-plus viruses are able to replicate on these cells. Virus yield from such an infection will be determined. If significant progeny viruses are produced in this infection, then the molecularly cloned fragment must contain the wild-type FHV TK gene.

DNA sequence analysis of this molecularly cloned sequence is used to reveal the presence in a precise location of the FHV TK gene by comparison with the known homologous TK proteins of other herpesviruses. A convenient restriction site within the gene is used for the subsequent molecular cloning of the FeLV envelope gene-containing expression cassette.

## B. FeLV Envelope Gene Expression Cassettes

The entire FeLV envelope gene (as described in Example 1) or a cassette containing the gp70 gene and a sequence encoding for an alternative transmembrane region (instead of FeLV p15E) is expressed by virtue of the juxtaposition of a strong promoter sequence directly upstream from the 5' end of the PstI-EcoRI restriction fragment containing the envelope gene or alternatively, the appropriate restriction fragment for an alternative construction. Promoters may be the HSV TK promoter, the RSV LTR promoter, the FHV TK promoter, the HSV α-4 gene promoter, or any of a variety of other strong promoters active within herpesvirus-infected cells. Additional strong FHV promoters useful to drive expression of the FeLV env gene in recombinant FHV can be identified by the examination of FHV-infected cells to determine FHV genes which are abundantly expressed during infection. It is anticipated that strong promoters driving the expression of some of the FHV structural genes, for example, those encoding the capsid proteins or the major envelope glycoproteins, will be found by such an examination. Such strong promoters could then be isolated and used to drive FeLV envelope gene expression. The in vitro plasmid constructions are routine and are performed using methods described by Maniatis et al. (supra).

These cassettes are inserted into the FHV TK gene at a convenient restriction site so as to interrupt the viral TK gene. This plasmid is used to insert this expression cassette into the FHV genome at the TK gene.

## C. In Vitro Recombination to Yield Live,
## Recombinant FHV Expressing FeLV Envelope Gene

The recombinant insertion vector containing the FELV envelope gene expression cassette (with p15E or with an alternative transmembrane region) within the FHV TK gene is used to co-transfect feline cells along with FHV genomic DNA, as in Example 1. Homologous recombination events between the plasmid and genomic TK gene sequences within transfected cells generate recombinant viruses containing the FeLV envelope gene cassette within the genomic TK gene. Progeny viruses from this transfection are harvested and allowed to replicate either in TK-minus feline cells in the presence of BUdR selection (see Example 1) or in wild-type TK-plus feline cells in the presence of the TK-specific drug araT; only TK-minus progeny viruses will be able to replicate efficiently in the presence of either of these drugs. Following the two rounds of selection, viruses are cloned and assayed for the presence and expression of FeLV envelope gene as described in Example 1.

Modifications of the above-described modes of carrying out the invention that are obvious to those of skill in the fields of recombinant DNA technology, immunology, veterinary medicine, and related fields are intended to be within the scope of the following claims.

## Claims

1. An insertion vector for use in transfecting mammalian cells to produce a recombinant form of a virus that encodes the expression of an immunogenic feline leukemia virus envelope protein, said vector comprising:
(a) a replicator;
(b) at least a portion of a nonessential region of the genome of said virus; and
(c) a chimeric gene inserted within said nonessential region comprising a promoter that is active in cells infected with the virus, a translation start codon, and a DNA sequence that codes for said protein and is under the control of said promoter.

2. An insertion vector as claimed in claim 1 wherein the nonessential region includes a marker function that permits selection of said recombinant form.

3. An insertion vector as claimed in claim 1 wherein the nonessential region comprises the thymidine kinase gene of said virus.

4. An insertion vector as claimed in any one of claims 1 to 3, wherein the virus is a virus that is capable of infecting cats.

5. An insertion vector as claimed in claim 4 wherein the virus is a feline-adapted virus or a vaccinia virus.

6. An insertion vector as claimed in claim 1 wherein (i) the virus is a vaccinia virus and the promoter is the vaccinia virus promoter; or (ii) the virus is feline herpesvirus and the promoter is a the herpes simplex I thymidine kinase promoter, the feline herpesvirus thymidine kinase promoter, the herpes simplex α-4 promoter or a feline herpesvirus capsid or major envelope glycoprotein promoter.

7. An insertion vector as claimed in any one of claims 1 to 6 wherein the immunogenic feline leukemia virus envelope protein encoded comprises FeLV gp85, and/or FeLV gp70 and a transmembrane region protein from a different source than FeLV p15E, e.g. the vesicular stomatitis G protein.

8. An insertion vector as claimed in any one of claims 1 to 6 wherein the immunogenic feline leukemia virus envelope protein is derived from FeLV subgroup A.

9. An infectious recombinant virus usable in a leukemia virus vaccine, said virus having a chimeric gene comprising a promoter that is active in cells to be infected with the virus, a translation start codon, and a DNA sequence that codes for an immunogenic feline leukemia virus envelope protein and is under the control of said promoter, said chimeric gene being inserted into a nonessential region of the genome of said virus.

10. A virus as claimed in claim 9 wherein the recombinant virus is feline-adapted, preferably a vaccinia virus.

11. A recombinant virus identifiable with ATCC No. VR 2215.

12. A feline leukemia virus vaccine comprising a sufficient amount of a infectious recombinant virus as claimed in any one of claims 9 to 11 to generate an immune response upon vaccinal infection and replication in the host, and a parenteral vehicle.

13. A feline leukemia virus vaccine comprising a parenteral vehicle and an immunogenically effective amount of an inactivated preparation of cells or cell fractions that had been infected with the recombinant virus as claimed in any one of claims 9 to 11.

14. A virus as claimed in any one of claims 9 to 11 for use in preventing disease in cats by vaccination thereof into cats.

15. The use of a vector as claimed in any one of claims 1 to 8 in producing a vaccine against feline leukemia virus.

16. An E. Coli K-12 strain indentifiable with ATCC No. 39533.

17. A method of producing an insertion vector for use in transfecting mammalian cells to produce a recombinant form of a virus that encodes the expression of an immunogenic feline leukemia virus envelope protein, which comprises providing, linked together,

    (a) a replicator;
    (b) at least a portion of a nonessential region of the genome of said virus; and
    (c) a chimeric gene inserted within said nonessential region comprising a promoter that is active in cells infected with the virus, a translation start codon, and a DNA sequence that codes for said protein and is under the control of said promoter.

18. A method of producing an infectious recombinant virus for use in a feline leukemia virus vaccine including the step of transfecting a cell with virus DNA and an insertion vector comprising

    (a) a replicator;
    (b) at least a portion of a nonessential region of the genome of said virus; and
    (c) a chimeric gene inserted within said nonessential region comprising a promoter that is active in cells infected with the virus, a translation start codon, and a DNA sequence that codes for said protein and is under the control of said promoter.

19. A method for making a feline leukemia virus vaccine which comprises formulating a virus having a chimeric gene comprising a promoter that is active in cells to be infected with the virus, a translation start codon, and a DNA sequence that codes for an immunogenic feline leukemia virus envelope protein and is under the control of said promoter, said chimeric gene being inserted into a nonessential region of the genome of said virus, and/or a cell carrying such a virus with a suitable carrier adjuvant or vehicle.

**GA-FeLV-B PROVIRUS**

pKHR-1

pGApv

**pGS20 VECTOR**

**pVGA INSERTION VECTOR**

VACCINIA TK GENE
VACCINIA PROMOTER
FeLV SEQUENCES

FIG. I

GA-FeLV B PROVIRAL GENOME

GA-FeLV B env GENE

FIG. 2

LTR gag | pol | env | LTR    GA FeLV B
                   |                    derived from
                 PvuII                  λ HF60

KmR    EcoRI    ApR

pKHR 1

ori

PvuII

PvuII
Ligation

LTR

EcoRI    ApR

pGApv

env

ori

PvuII

FIG. 3

GENERATION OF RECOMBINANT VACCINIA
VIRUS CONTAINING FELV *env* GENE

INFECTION ← WILD TYPE VACCINIA VIRUS

VACCINIA INFECTED CELL

TRANSFECTION ← pVGA

env

P ATG

pVGA

P env

X X

*IN VIVO* HOMOLOGOUS RECOMBINATION

VACCINIA VIRUS DNA

TK

RECOMBINANT VACCINIA VIRUS DNA

P env

RECOMBINANT VACCINIA VIRUS v FeLV *env*

PROGENY VIRUS →

BUdR SELECTION 143 (tk⁻) CELLS

FIG. 4

**vFeLVenv - ENCODED EXPRESSION**

**OF FeLVenv GENE**

← 92Kd

← 66Kd

150μg  250μg 250μg     PURIFIED
     CELL-MEMBRANE       VIRUS
      PREPARATION

FIG. 5

## FIG. 6A    vFeLV *env* : FIXED-CELL STAINING

## FIG. 6B    vFeLV *env* : SURFACE STAINING

## FIG. 7A
**TOTAL CELL LYSATE IMMUNOPRECIPITATION**

1  2  3  4

← 92Kd

← 66Kd

## FIG. 7B
**SURFACE IMMUNOPRECIPITATION**

1  2  3  4

← 92Kd

← 66Kd

FIG. 8

FIG. 9

*A*

EcoRI — Hind III

VSV G

EcoRI

*B*

MstII

FeLV gp70
(C-terminus)

Hind III

3' tk

MstII

FeLV gp70
(N-terminus)

promoter

5' tk

*C*

Amp^R
E. coli ori

pVGA-VSVG

☐ FeLV env gene

▨ VSV transmembrane domain

▦ vaccinia tk gene

▦ vaccinia 7.5 kd promoter

FIG. 10